# EUROPEAN PATENT APPLICATION

(11) **EP 4 378 435 A1**
(43) Date of publication of application: **05.06.2024**
(21) Application number: 22211181.7
(22) Date of filing: 02.12.2022
(51) Int. Cl.: A61F 13/00, A61N 1/00

(54) **WOUND DRESSING WITH REDUCED MICROBIOLOGICAL GROWTH**

(71) Applicant: EPFF Electrical Pipe For Fluid transport AB, 736 32 Kungsör (SE)
(72) Inventor: ANDERSSON, Thomas, 736 32 KUNGSÖR (SE)
(74) Representative: Brann AB

(57) **Abstract**

A wound dressing (12) and related systems and methods are proposed. The wound dressing (12) comprises: a first electrode (18), a second electrode (20), a first layer (16) for placement on top of a wound, and an electric interface (22). The first electrode (18) contacts the first layer (16), the second electrode (20) contacts the first layer (16) and is spaced apart from the first electrode (18). The first layer (16) is electrically conductive and constitutes an uninterrupted resistive conductor between the first electrode (18) and the second electrode (20). The electric interface (22) is coupled to the first electrode (18) and the second electrode (20) and configured to operationally couple to an electric power source (14) for generating an electric current between the first electrode (18) and the second electrode (20) via the first layer (16).

## Description

### Technical field

The proposed technology relates generally to the field of wound dressings. The proposed technology relates specifically to the prevention of microbiological growth on or in wound dressings.

### Background

Wound dressings, or medical dressings, are applied to wounds to promote healing and protect wounds from further harm. There are many types of dressings. They can be classified as primary dressings that are applied directly to wounds or lesions and secondary dressings that are needed to secure a primary dressing. Both types can have therapeutic or protective functions.

Wound dressings are also classified as passive or interactive. Passive dressings typically have the main function of protecting or covering the wound. It is common to use them as secondary dressings or as primary dressings for wounds with mild exudate levels. Passive dressings are typically non-occlusive, which means that they do not act as a barrier for bacteria. Thus, bacteria may enter the passive dressing from the outside and proliferate within the dressing. Interactive dressings are typically used to promote healing. This type of dressings is semi-occlusive or occlusive. A semi-occlusive dressing allows water vapor, oxygen, and carbon dioxide to pass through the dressing and at the same time acts as a barrier for bacteria. An occlusive dressing prevents passage of gases, liquids, and bacteria. In both types, a moist environment is maintained at the wound. This typically promotes healing but can also be suitable for bacterial growth.

### Object

Microbiological growth in a wound dressing can negatively affect the healing of a wound. Thus, it is an object of the proposed technology to inhibit bacterial and other microbiological growth in a wound dressing.

### Summary

In a first aspect of the proposed technology, a wound dressing is proposed for covering a wound, wherein the wound dressing comprises: a first electrode, a second electrode, a first layer for placement on top of a wound, and an electric interface. The first electrode contacts the first layer, the second electrode contacts the first layer and is spaced apart from the first electrode. The first layer is electrically conductive and constitutes an uninterrupted resistive conductor between the first electrode and the second electrode. The electric interface is coupled to the first electrode and the second electrode and configured to operationally couple to an electric power source for generating an electric current between the first electrode and the second electrode via the first layer. Worded differently, the electric interface is configured to integrate the first electrode, the first layer, and the second electrode in a closed electric circuit powered by an electric power source.

It is understood that the electric interface is configured to be electrically coupled to an electric power source. It may be configured for generating an electric current between the first electrode and the second electrode via the first layer.

The first layer is electrically conductive and constitutes an uninterrupted resistive conductor between the first electrode and the second electrode, and contributes to reduce, or prevent, electrolysis in or at the first layer, for example in wound exudate absorbed by the first layer.

In a second aspect of the proposed technology, a wound dressing system is proposed. The wound dressing system comprises: a wound dressing according to the first aspect of the proposed technology, and an electric power source operationally coupled to the electric interface and configured for generating an electric current between the first electrode and the second electrode via the first layer for inhibiting, or reducing, microbiological, or bacterial, growth in, and/or on, the first layer.

In a third aspect of the proposed technology, a method is proposed for reducing microbiological growth in and/or on a wound dressing according to the first aspect of the proposed technology. The method comprises: generating an electric current in the first layer, for example by an electric power source operationally coupled to the electric interface.

In a fourth aspect of the proposed technology, a method is proposed for treatment of a wound using the wound dressing according to the first aspect of the proposed technology. The method comprises: placing the wound dressing on top of the wound, and generating an electric current in the first layer, for example by an electric power source operationally coupled to the electric interface.

Here, a wound is understood to encompass a disruption in the continuity of the epithelial lining of the skin or mucosa, for example resulting from physical, thermal, or radiological damage.

The first layer may be continuous. This means that the first layer has no disconnected partitions. The first layer may have a circumferential outer edge. The circumferential outer edge may be rectangular, circular or have a shape adapted for placement over a wound on a particular body part, such as an elbow or a knee. Worded differently, the circumferential outer edge may be shaped to conform to a wound on a particular body part.

The first layer may define a wound area for covering the wound. The wound area may have a first side and an opposite second side. The first electrode may be positioned at the first side and the second electrode may be positioned at the second side. Worded differently, the first layer has a wound area positioned between the first electrode and the second electrode, or the first electrode and the second electrode may define a wound area between them.

The first layer may be inert to an electric current passing through the first layer. It is understood that the electric current is for inhibiting, or reducing, microbiological growth. This means that structural or chemical properties of the first layer as such are unchanged by the electrical current passing through first layer. It also means that no chemicals or substances are released from first layer by the electric current.

The wound dressing, the first layer may be elastic, or the wound area of the first layer may be elastic. This allows for the wound dressing to better conform to a wound.

It is specified above that the wound dressing comprises a first electrode and a second electrode, that the first electrode contacts the first layer, and that the second electrode contacts the first layer and is spaced apart from the first electrode.

The first electrode may be elongated and extend in a first direction relative to the wound area. The second electrode may also be elongated and extend in the first direction relative to the wound area. Worded differently, the first electrode may be elongated, the second electrode may be elongated, and the first electrode and the second electrode may be aligned. For example, each of the first electrode and the second electrode may be a thin metal strip. Each of the first electrode and the second electrode may be straight. The first electrode and the second electrode may be parallel. The first electrode and the second electrode may be abreast. Taken together, this means that the wound area between the first electrode and the second electrode is rectangular, with the first electrode and the second electrode positioned at opposite sides of the rectangle. The features specified here contribute to distribute the electric current over the wound area.

The first layer may have an inner surface and an outer surface. It is understood that the inner surface is intended to face the wound and the outer surface is intended to face away from the wound. The first electrode and the second electrode may contact, or connect to, the outer surface of the first layer. This is advantageous in multi-layer wound dressings. Alternatively, the first and second electrodes may be embedded in the first layer between the inner surface and the outer surface. This means that the electrodes are covered by the material of the first layer and that they are spaced apart from the inner surface and the outer surface. This is advantageous in single-layer wound dressings.

The first layer may be a single layer. Alternatively, the first layer may be composed of several overlapping, contacting, laminated, or sandwiched electrically conductive layers. The wound dressing may be a single-layer dressing. This means that the first layer is the only layer of the wound dressing.

As described above, the first layer may have a circumferential outer edge. The first layer be adhesive only at the outer edge. Alternatively, the complete first layer, or the complete inner surface of the first layer, may be adhesive. Throughout these specifications, adhesive properties are understood to mean the fastening of the wound dressing on a patient. Worded differently, if it is specified that a layer, or area, is adhesive, it is understood that it is configured for fastening the wound dressing, for example at the wound.

Alternatively, the complete first layer, or the complete inner surface of the first layer, may be non-adhesive. This means that a dressing support, for example medical tape, is required for fastening, or securing, the wound dressing.

The single-layer dressing may be a passive dressing. A passive dressing is understood to be configured to dehydrate, or drain, a wound. It is further understood that a passive dressing may be configured to become hydrated by the wound.

The single-layer dressing may be a non-occlusive dressing, such as a gauze or tulle dressing. A non-occlusive dressing is here understood as a dressing configured to allow passage of liquids and gases, or air. The first layer may be non-occlusive, which means that it allows passage of liquids and gases, or air. It is understood that a non-occlusive dressing or layer may allow passage of bacteria from outside the dressing or layer to a wound.

Non-occlusive dressings are commonly used for wounds with high exudates levels or moderate bleeding. The first layer of the non-occlusive and single-layer dressing may comprise woven or non-woven fibers, such as cotton or rayon fibers. The first layer, or the woven or non-woven cotton or rayon, of the non-occlusive dressing may comprise carbon fibers arranged for making the first layer electrically conductive. The first layer of the non-occlusive dressing may comprise a polymer. The polymer as a such may be electrically conductive. Alternatively, the polymer may be an electrically insulating polymer, such as a polyester, and it may comprise an additive, such as carbon black or carbon fibers, for making the first layer of the non-occlusive dressing electrically conductive.

The non-occlusive wound dressing may be a foam dressing. The first layer may comprise, or be formed from, a foam material. The foam material may be elastic, thus allowing the area or shape of the first layer to be changed when used. The foam material may comprise a polymer, such as a polyurethane foam. The polymer may be electrically insulating. The foam material may comprise an additive, such as carbon black or carbon fibers, for making the first layer of the non-occlusive dressing electrically conductive. The foam layer may be a 3D foam layer, comprising a 3D structure with channels arranged in the foam layer for promoting dehydration, or draining, of exudate from the wound.

The non-occlusive single-layer dressing may be a primary dressing. A primary dressing is here understood to be a dressing that is configured or intended to be in direct contact with a wound. The proposed technology then has the advantage of an improved inhibition of microbiological growth at the wound. The first layer may be configured, or arranged to, contact the wound.

The single-layer dressing may be a secondary dressing for supporting a primary dressing at the wound. It is understood that the primary dressing is positioned between the secondary dressing and the wound. This means that the secondary dressing is spaced apart from the wound. However, bacteria may migrate from the secondary dressing to the primary dressing, for example if the secondary dressing is non-occlusive and if it becomes wet from an external source, or if the wound exudate level is high and the exudate enters the secondary dressing. The wound dressing being a secondary dressing allows for higher currents and voltages across the first layer.

The single-layer dressing may be an interactive, or active, dressing. An interactive dressing is understood to be configured to provide a moist environment at the wound, or to prevent a wound from drying up. An interactive dressing may be configured to promote healing of the wound.

The first layer may be configured to maintain a moist environment at the wound. The first layer may be non-absorbent and/or hydrophobic. Alternatively, the first layer may be absorbent and/or hydrophilic.

The single-layer dressing may be a semi-occlusive dressing or an occlusive dressing. A semi-occlusive, or semipermeable dressing, is here understood as a dressing configured to prevent passage of liquids and to allow passage of gases, or air. Semi-occlusive dressings are commonly used for superficial or shallow wounds with low exudates levels. An occlusive dressing, or non-permeable dressing, is understood as a dressing configured to prevent passage of liquids and gases, or air.

The first layer may be semi-occlusive, which means that it prevents passage of liquids and allows passage of gases, or air. This contributes to make the dressing semi-occlusive. Alternatively, the first layer may be occlusive, which means that it prevents passage of both liquids and gases, or air. This contributes to make the dressing occlusive. It is understood that semi-occlusive and occlusive dressing prevent passage of bacteria from outside the dressing to a wound.

The wound dressing may be a non-absorptive film dressing. The first layer of the semi-occlusive single-layer dressing may comprise, or constitute, a non-absorptive film, such as polymer film. Here, a non-absorptive property is understood to relate to exudate from the wound or water. It is understood that the film must be sufficiently thin for the dressing, or first layer, to be semi-occlusive. The film may be elastic, thus allowing the area or shape of the first layer to be changed when used. The film may be an of an electrically insulating polymer, such as polyurethane, and it may comprise an additive, such as carbon black or carbon fibers, for making the first layer of the semi-occlusive dressing electrically conductive.

Alternatively, the semi-occlusive wound dressing may be a foam dressing. The first layer may comprise, or be formed from, an absorptive foam material. Here, an absorptive property is understood to relate to exudate from the wound or water. The foam material may comprise a polymer, such as a polyurethane. The polymer may be electrically insulating, and the foam material may comprise an additive, such as carbon black or carbon fibers, for making the first layer of the non-occlusive dressing electrically conductive.

The occlusive single-layer dressing may be a primary dressing. As defined above, a primary dressing is understood to be a dressing that is configured or intended to be in direct contact with a wound. The first layer may be configured, or arranged to, contact the wound.

The wound dressing may be a composite dressing. A composite dressing is understood as a dressing having multiple physiologically or functionally distinct layers. It is understood that the first layer of the composite dressing may comprise any of the features of the first layer described above in relation to the single-layer dressing.

In addition to the first layer, the wound dressing may comprise an outer additional layer. The outer additional layer may conform to, or cover, the abovementioned outer surface of the first layer. It is understood that the outer additional layer may extend from the first layer and that it may be configured to conform to the area around the wound. The area of the first layer may be smaller than the area of the outer additional layer. It is further understood that the outer additional layer is positioned outside the first layer relative to the wound. The outer additional layer may be in direct contact with the first layer. The outer additional layer may be attached to, or supported by, the first layer.

The outer additional layer may be electrically insulating. For example, it may comprise an electrically insulating polymer, such as polyurethane. This contributes to prevent an electric current from deviating from its intended path in between the first electrode and the second electrode, for example by electrically grounded equipment contacting the wound dressing.

The outer additional layer may be a backing layer configured to support the first layer relative to the wound. For example, the backing layer may form an adhesive boundary around the first layer, or an adhesive surface outside the circumferential outer edge of the first layer. Worded differently the backing layer may be adhesive. The backing layer is advantageous in combination with a non-adhesive first layer configured to contact the wound or area around the wound. For example, the backing layer may be an adhesive fabric tape or a polyurethane film, or it may be composed of coated paper. A backing layer is understood to be the outermost layer of wound dressing.

Alternatively, the outer additional layer may be non-adhesive. This means that a dressing support, for example medical tape, is required for fastening, or securing, the wound dressing.

The composite dressing may be a passive dressing. The composite dressing may be a non-occlusive dressing. As described above, the first layer may be non-occlusive. Additionally, the outer additional layer may be non-occlusive, which means that the composite dressing allows passage of liquids and gases, or air. The outer additional layer may comprise woven or nonwoven fabric. For example, the fabric may be of cotton or polyester. Alternatively, the outer additional layer may be of a foam material.

The composite dressing may be a primary dressing. Alternatively, the composite dressing may be a secondary dressing for supporting a primary dressing at the wound.

The composite dressing may be an interactive, or active, dressing. As described above, the first layer may be configured to maintain a moist environment at the wound.

The composite dressing may be a semi-occlusive dressing or an occlusive dressing.

As described above, the first layer may be non-occlusive or semi-occlusive. The outer additional layer may be semi-occlusive or occlusive. This contributes to make the dressing semi-occlusive or occlusive.

The outer additional layer may comprise, or constitute, a non-absorptive film, such as polyurethane film. It is understood that the film must be sufficiently thin for the dressing, or the outer additional layer, to be semi-occlusive, and that the film may be electrically insulating.

As described above, the first layer may comprise, or be formed from, an absorptive foam material. Worded differently, the first layer may be a hydrophilic foam. The outer additional layer may comprise a non-absorptive foam material. Worded differently, the first layer may be a hydrophilic foam. For example, foam material of the outer additional layer may comprise a polymer, such as a silicone-rubber foam. It is understood that the foam material of the outer additional layer may be electrically insulating.

In addition to the first layer, or in addition to the first layer and the outer additional layer, the wound dressing may comprise an inner additional layer. The inner additional layer may conform to, or cover, the abovementioned inner surface of the first layer. It is understood that the first layer may extend from the inner additional layer and that the inner additional layer may be configured to conform to the area of the wound. The area of the inner additional layer may be smaller than the area of the first layer. It is further understood that the inner additional layer is positioned inside the first layer relative to the wound. This means that first layer is spaced apart from the wound. The inner additional layer may be in direct contact with the first layer. The inner additional layer may be attached to, or supported by, the first layer. The inner additional layer may be configured, or arranged to, contact the wound.

The inner additional layer may be electrically insulating. For example, it may comprise an electrically insulating polymer, such as polyurethane. The first layer is spaced apart from the wound, which allows for higher currents and voltages across the first layer.

Alternatively, the inner additional layer may be electrically conductive. This contributes to inhibition of microbiological growth at the wound.

The complete inner additional layer may be adhesive. Alternatively, the complete inner additional layer may be non-adhesive. Worded differently, the inner additional layer may be configured to not adhere or stick to the wound, for example to granulating tissues.

The composite dressing may be a passive dressing. The composite dressing may be a non-occlusive dressing. As described above, the first layer and the outer additional layer, if present, may be non-occlusive. Additionally, the inner additional layer may be non-occlusive, which means that the composite dressing allows passage of liquids and gases, or air. For example, the inner additional layer may be a coating on the first layer, such as a porous-polymer coating. This is advantageous in combination with the first layer comprising woven and non-woven cotton fibers.

The coating may be electrically conductive. For example, the coating may comprise carbon black. This contributes to a reduced microbiological growth at the wound.

The composite dressing may be a primary dressing or intended to be used as a primary dressing. Alternatively, the composite dressing may be a secondary dressing or intended to be used as a secondary dressing for supporting a primary dressing at the wound. The composite dressing may be an interactive, or active, dressing. As described above, the first layer may be configured to maintain a moist environment at the wound. The proposed technology then has the advantage of an improved inhibition of microbiological growth at the wound. The first layer may be configured, or arranged to, contact the wound.

The composite dressing may be a semi-occlusive dressing or an occlusive dressing. As described above, the first layer may be non-occlusive, semi-occlusive, or occlusive. The inner additional layer may be non-occlusive or semi-occlusive.

The inner additional layer may comprise, or constitute, a non-absorptive film, such as polyurethane film. It is understood that the film must be sufficiently thin for the dressing, or the inner additional layer, to be semi-occlusive, and that the film may be electrically insulating. This allows for higher currents and voltages to be used in the first layer.

As described above, the first layer may comprise, or be formed from, an absorptive foam material. Worded differently, the first layer may be a hydrophilic foam. The inner additional layer may comprise a non-absorptive foam material. Worded differently, the first layer may be a hydrophilic foam. For example, foam material of the inner additional layer may comprise a polymer, such as a silicone-rubber foam. It is understood that the foam material of the inner additional layer may be electrically insulating. This allows for higher currents and voltages to be used in the first layer.

It is specified above that the electric interface is coupled to the first electrode and the second electrode. In the wound dressing the electric interface is configured to be operationally coupled to an electric power source. In the system the electric power source is operationally coupled to the electric contact.

The electric interface may be supported by the first layer. This is particularly advantageous in a single-layer dressing. The electric interface may be supported by the abovementioned outer additional layer or backing layer. This is advantageous if the outer additional layer or backing layer are adhesive. If the electric interface is supported by the outer additional layer or backing layer, it may be spaced apart tangentially, or horizontally, relative to the first layer. This means that the electric interface is positioned beside the first layer when the wound dressing is applied at a wound.

The electric power source may be external to, or separate from, the wound dressing.

The electric interface may be configured for a resistive, or wired, power transfer from the electric power source. This is suitable for both an alternating and a direct current in the first layer.

The electric interface may comprise a first fixed connector part and a second fixed connector part, wherein each of the first fixed connector part and the second fixed connector part is a single-terminal connector part. It is understood that the first fixed connector part and the second fixed connector part are fixed to the wound dressing. It is further understood that a single-terminal connector part has a single terminal. A terminal is understood as the endpoint of an electrical conductor. It is understood that the first and second fixed connector parts are configured to integrate the first electrode, the first layer, and the second electrode in a closed electric circuit.

A connector is here understood to have a fixed connector part and a detachable connector part. This means that the fixed connector part may be configured to mate with a detachable connector part.

The first fixed connector part, or the terminal of the first fixed connector part, may be coupled to the first electrode, for example by a first lead. Similarly, the second fixed connector part, or the terminal of the second fixed connector part, may be coupled to the second electrode, for example by a second lead. It is understood that the first lead and the second lead may be electrically insulated from the first layer. The first and second leads allows for the first and second fixed connector parts to be spaced apart from the above-mentioned wound area. The first and second leads may be flexible, thus allowing for the wound dressing to conform to the shape of the body at the wound.

The first and second fixed connector parts may be spaced apart from the above-mentioned wound area. For example, the first and second fixed connector parts may be spaced apart from the wound area by at least 1 cm, 2 cm, or 3 cm. This allows for the fixed connector parts to be manipulated with little interference with the wound.

The first and second fixed connector parts may each form part of a snap connector, or snap mount. For example, the terminal of each of the first and second fixed connector parts may be the male part of a snap connector. A snap connector is understood as a connector for which a pushing force must be overcome for mating the detachable connector part to the fixed connector part and a pulling force must be overcome for detaching the detachable connector part from the fixed connector part. The fixed connector parts being spaced apart from the wound area is particularly advantageous in combination with the snap mount to reduce the effect of force required for mating the parts of the snap mount on the wound. Alternatively, the first and second fixed connector parts may each form part of a bayonet connector, or bayonet mount. This contributes to reduce the lateral force relative to the patient when connecting the first and second fixed connector parts. The first and second fixed connector parts may each be configured to cooperate with a manually openable springloaded clamp or clip with, such as a crocodile clip. This also contributes to reduce lateral forces relative to the patient when connecting.

The first fixed connector part and the second fixed connector part may be positioned at opposite sides of the wound area. This contributes to distribute the load from wiring connecting the wound dressing to an electric power source, which in extension contributes to reduce the load on the wound as such.

The system may comprise a first detachable connector part and a second detachable connector part. The first detachable connector part and the second detachable connector part are respectively operationally connected to, or configured to be operationally connected to, the first fixed connector part and the second fixed connector part. Worded differently, the first detachable connector part and the second detachable connector may operationally couple, or be configured to operationally couple, the electric power source to the electric interface, for example by first and second electric conduits that respective connects the first detachable connector part and the second detachable connector to the electric power source.

The electric interface may comprise a single fixed connector part, wherein the single fixed connector part is a multi-terminal connector part. The multi-terminal connector part may be a two-terminal connector part. It is understood that a two-terminal connector part has a first terminal and a second terminal. It is understood that the connector part is configured to integrate the first electrode, the first layer, and the second electrode in a closed electric circuit.

The fixed connector part, or the first terminal of the fixed connector part, may be coupled to the first electrode, for example by a first lead. Additionally, the fixed connector part, or the second terminal of the fixed connector part, may be coupled to the second electrode, for example by a second lead. As described above, the first and second leads may be electrically insulated from the first layer. The first and second leads may be flexible, thus allowing for the wound dressing to conform to the shape of the body at the wound. The first and second leads may be in the form of an electrically insulated wire.

The fixed connector part may be spaced apart from the above-mentioned wound area. For example, it may be spaced apart from the wound area by at least 1 cm, 2 cm, or 3 cm. This allows for the electric interface to be manipulated with little interference with the wound.

The fixed connector part may form part of a bayonet connector, or bayonet mount.

In comparison with the pair of single-terminal fixed connector parts, the multi-terminal connector part collects the weight of wiring to a small area, which may have the disadvantage of a greater load on the wound. However, it has the advantage of an easier handling when connecting wiring to the electric power source.

The system may comprise a detachable connector part. The detachable connector part is operationally connected to, or configured to be operationally connected to, the fixed connector part. Worded differently, the detachable connector part may operationally couple, or be configured to operationally couple, the electric power source to the electric interface, for example by first and second electric conduits that respectively connects the detachable connector part to the electric power source.

The electric interface may be configured for a wireless power transfer from the electric power source. This is suitable for an alternating current in the first layer.

The electric interface may comprise a receiver for wireless electric power transfer. The first electrode and the second electrode may form part of, or constitute, the receiver. Alternatively, the receiver may be coupled to the first electrode and the second electrode, for example by a first lead and a second lead. This way, the receiver is hard wired to the first layer. The receiver may be electrically insulated from the first layer. It is understood that the first lead and the second lead may be electrically insulated from the first layer. It is understood that the receiver may be configured to generate an electric current in the first layer by coupling to a time-varying electromagnetic field. It is further understood that the receiver may integrate the first electrode, the first layer, and the second electrode in the closed electric circuit.

The receiver may be supported by, embedded in, or aligned with the first layer, or the outer additional layer or backing layer. The receiver may be positioned between the first electrode and the second electrode, or in the wound area.

The receiver may be configured to be capacitively coupled to the electric power source. The receiver may comprise a first receiver electrode coupled to the first electrode, for example by the first lead. Alternatively, the first electrode may constitute a first receiver electrode of the receiver. The receiver may comprise a second receiver electrode coupled to the second electrode, for example by the second lead. Alternatively, the second electrode may constitute a second receiver electrode of the receiver.

The first receiver electrode and the second receiver electrode may be supported by, embedded in, or aligned with the first layer. The first receiver electrode and the second receiver electrode may be flexible. For example, each may be formed by a thin metal sheath. This allows for the first layer to conform to the area at the wound also when the first layer supports the first and second receiver electrodes.

It is understood that a receiver electrode is configured for capacitive power transfer, or to couple to a varying electric field. Here, it is understood that the electric field is an electromagnetic field dominated by an electric dipole. A capacitive power transfer is understood as a coupling in a circuit.

The system may comprise a first transmitter electrode coupled to the electric power source, for example by a first electric cable. The system may further comprise a second transmitter electrode coupled to the electric power source, for example by a second electric cable. This way, the electric power source can wirelessly generate an electric current in the first layer. It is understood that a transmitter electrode is configured to cooperate with, or capacitively couple to, a receiver electrode, for wireless, or capacitive, power transfer. It is understood that a transmitter electrode is configured to generate a time-varying electric field.

The receiver may be configured to inductively couple to the electric power source. The receiver may comprise a receiver coil coupled to the first electrode and the second electrode, for example by the first lead and the second lead. The receiver coil may be supported by, embedded in, or aligned with the first layer. The receiver coil may be flexible. This allows for the first layer to conform to the area at the wound also when the first layer supports the receiver coil. It is understood that a receiver coil is configured for inductive transfer, or to couple to a varying magnetic field. Here, an inductive power transfer is understood as a coupling in a circuit.

The system may comprise a transmitter coil coupled to the electric power source, for example by two electric conduits. This way, the electric power source can wirelessly generate an electric current in the first layer. It is understood that a transmitter coil is configured to cooperate with, or couple to, a receiver coil, for inductive power transfer. It is understood that a transmitter coil is configured to generate a time-varying magnetic field.

The receiver may be configured to electromagnetically couple to the electric power source. The receiver may comprise a receiver antenna coupled to the first electrode and the second electrode, for example by the first lead and the second lead. The receiver antenna may be supported by, embedded in, or aligned with the first layer. The receiver antenna may be flexible. This allows for the first layer to conform to the area at the wound also when the first layer supports the receiver antenna. It is understood that a receiver antenna is configured for radio-power transfer, or to couple to a varying electromagnetic field. Here, a radio-power transfer is understood as a coupling in a circuit.

The system may comprise a transmitter antenna coupled to the electric power source, for example by two electric conduits. This way, the electric power source can wirelessly generate an electric current in the first layer. It is understood that a transmitter antenna is configured to cooperate with, or couple to, a receiver antenna, for radio-power transfer. It is understood that a transmitter antenna is configured to generate a time-varying electromagnetic field.

The electric power source may be configured to be powered by mains electricity, or mains utility power. Alternatively, the electric power source may comprise a battery carrying stored energy.

It is specified above that the electric power source may be external to, or separate from, the wound dressing. Alternatively, the wound dressing may comprise the electric power source. The electric power source may be supported, or fixed, relative to the first layer. The electric power source may be supported by the abovementioned outer additional layer or backing layer.

It is specified above that the wound dressing comprises an electric interface, and that the electric interface is configured to operationally couple to an electric power source, or that it is configured to integrate the first electrode, the first layer, and the second electrode in a closed electric circuit powered by an electric power source. The first and second electrodes may form, or constitute, the electric interface, or the electric interface may be structurally integrated with the first and second electrodes. This means that there are no leads connecting an electric interface to the electrodes, as described above. For example, the first electrode may form, or constitute, the first fixed connector part or the first receiver electrode, and the second electrode may form, or constitute, the second fixed connector part or the second receiver electrode. The above means that, the electric power source is coupled to the first electrode and the second electrode and configured for generating an electric current between the first electrode and the second electrode via the first layer. Worded differently, the electric power source, the first electrode, the first layer, and the second electrode may form part of, or be integrated in, a closed electric circuit, wherein the electric power source is configured to generate an electric current in the first layer.

The electric current in the first layer may be in the range 0.001-1 mA, 0.001-0.01 mA, or 0.01-1 mA. Preferably, the current is below 1 mA, or 0.1 mA. Preferably, the current is greater than 0.001 mA, or 0.01 mA. The electric current may be generated at an electric potential over the first layer, or between the first electrode and the second electrode, in the range of 0.01-5 V, or 0.1-3 V. The electric potential may be below 5 V, 3 V, or 1V. The electric potential may greater than 0.01 V, or 0.1 V. These currents and voltages are advantageous if the first layer is in direct contact with the wound.

The electric current in the first layer may be in the range 0.01-10 mA, 0.01-0.1 mA, or 0.1-10 mA. Preferably, the current is below 10 mA, or 1 mA. Preferably, the current is greater than 0.01 mA, or 0.1 mA. The electric current may be generated at an electric potential over the first layer, or between the first electrode and the second electrode, in the range of 0.1-50 V, or 1-30 V. The electric potential may be below 50 V, 30 V, or 10V. The electric potential may greater than 0.1 V, 1 V, 3V, or 10 V. These currents and voltages are advantageous if the first layer is not in direct contact with the wound.

It is understood that the electric power source is configured to generate the electric potential. It is understood that other components may influence the potential, for example the electric interface.

The first electric current may be a direct current. Alternatively, the first electric current may be an alternating current. The alternating current may have a frequency between 0.5 kHz and 10 kHz, or between 1 kHz and 5 kHz.

The electric power source may be configured for supplying a pulsed, or intermittent, electric current. The pulses may have a combined pulse length over a period that is equal to or less than 50%, less than 10%, or less than 1% of the length of the period. This is advantageous in combination with the electric interface being configured for wired power transfer from the electric power source since it allows for an unsupervised application of the technology.

### Brief description of the drawings

A more complete understanding of the abovementioned and other features and advantages of the proposed technology will be apparent from the following detailed description of different embodiments in conjunction with the appended schematic drawings, wherein:
Fig. 1 illustrates an embodiment of a wound dressing system,
Fig. 2 illustrates another embodiment of a wound dressing system,
Fig. 3 illustrates another embodiment of a wound dressing system,
Fig. 4 illustrates another embodiment of a wound dressing system,
Fig. 5 illustrates another embodiment of a wound dressing system,
Fig. 6 illustrates another embodiment of a wound dressing system,
Fig. 7 illustrates an alternative wound dressing for the wounds systems of Fig. 1.
Fig. 8 illustrates an alternative wound dressing for the wounds systems of Fig. 3.
Figs. 9a to 9e illustrate different embodiments of wounds dressing for wired power transfer, and
Figs. 10a to 10e illustrate wound dressings corresponding to those of Figs. 9a to 9e for wireless power transfer.

### Description of the drawings

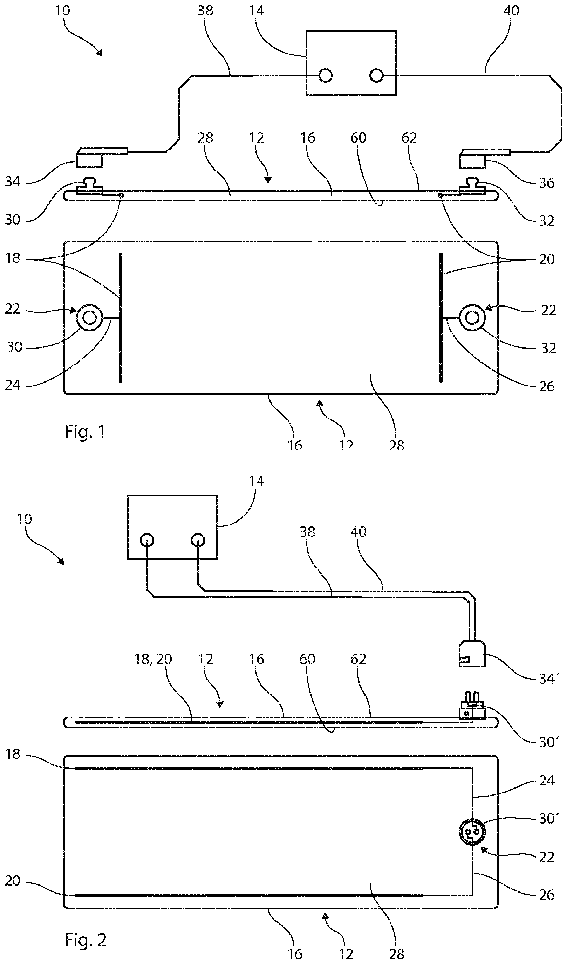

A wound dressing system 10 with a wound dressing 12 and an electric power source 14 is illustrated in Fig. 1. The wound dressing 12 is shown both in a side view and a top view. The electric power source 14 is external to and separate from the wound dressing 12.

The wound dressing 12 has a continuous and electrically conductive first layer 16 intended to be placed on top of a wound. It further has a first electrode 18 and a second electrode 20 that are spaced apart and contact the first layer 16. This way, the first layer 16 constitutes an uninterrupted electric conduit, or resistive conductor, between the first electrode 18 and the second electrode 20. The first and second electrodes 18 and 20 are thin metal strips that are straight, parallel, of the same length, and abreast, thus defining a rectangular wound area 28 between them intended to cover a wound.

The first layer has an inner surface 60 and an opposite outer surface 62. The inner surface 60 is intended to face a wound. The first electrode 18 and the second electrode 20 are embedded in the first layer 16 between the inner and outer surfaces 60 and 62.

The wound dressing 12 further has an electric interface 22 that is coupled to the first electrode 18 and the second electrode 18 by a flexible first lead 24 respective a flexible second lead 26. The first and second leads 24 and 26 are electrically insulated from the first layer 16. The electric interface 22 is attached to and supported by the first layer 16.

The electric interface 22 has a first fixed connector part 30 and a second fixed connector part 32 fixed to the first layer 16. The first fixed connector part 30 and the second fixed connector part 32 are both single-terminal connector parts. The terminal of the first fixed connector part 30 is coupled to the first electrode 18 by the first lead 24, and the terminal of the second fixed connector part 32 is coupled to the second electrode 30 by the second lead 26. The first and second fixed connector parts 30 and 32 are spaced apart from the above-mentioned wound area 28 by the first and second leads 24 and 26. The first fixed connector part 30 and the second fixed connector part 32 are positioned at opposite sides of the wound area 28.

The system 10 has a first detachable connector part 34 and a second detachable connector part 36 that are respectively coupled to the electric power source 14 by a first electric conduit 38 and a second electric conduit 40 in the form of electric wires. In operation, the first detachable connector part 34 and the second detachable connector part 36 are respectively connected to the first fixed connector part 30 and the second fixed connector part 32. In Fig. 1, the first detachable connector part 32 and the second detachable connector part 34 are illustrated as disconnected.

The first and second fixed connector parts 30 and 32 are male members of a snap connector, and the first detachable connector part 34 and the second detachable connector part 36 are cooperating female members of the snap connector.

This way, the electric interface is operationally coupled to the electric power source 14. The electric interface 22 also integrate the first electrode 18, the first layer 16, and the second electrode 20 in a closed electric circuit powered by an electric power source 14. When activated, the electric power source 14 generates an electric current in the first layer 16 between the first electrode 18 and the second electrode 20, which inhibits microbiological growth in or on the first layer 16.

Another wound dressing system 10 is illustrated in Fig. 2. The topology and components of this wound dressing system 10 differs from the wound dressing system 10 of Fig. 1 by the electric interface 22 having a single fixed connector part 30' fixed to the first layer 16. The fixed connector part 30' is a two-terminal connector part. One of the terminals is coupled to the first electrode 18 by the first lead 24 and the other terminal is coupled to the second electrode 30 by the second lead 26.

The fixed connector part 30' is spaced apart from the wound area 28 by the first and second leads 24 and 26.

The system 10 has a detachable connector part 34' coupled to the electric power source 14 by a first electric conduit 38 and a second electric conduit 40 in the form of electric wires. In operation, the detachable connector part 34' is connected to the fixed connector part 30'. In Fig. 2, the detachable connector part 34' is illustrated as disconnected.

The fixed connector parts 30' is a male member of a bayonet connector, and the detachable connector part 34' is a cooperating female member of the bayonet connector.

In the embodiments of Figs. 1 and 2, the electric power source 14 is powered by mains electricity. It can supply a direct current to the first layer 16 that is in the range 0.01-10 mA at a potential difference of 0.1-50 V between the first electrode 18 and the second electrode 20. It supplies the electric current intermittently with an integrated pulse length that is less than 1% of the operation time. In an alternative embodiment, it can supply an alternating current to the first layer 16 that is in the range 0.001-1 mA between the first electrode and the second electrode and at a frequency in the range 0.5 kHz and 10 kHz. It supplies the electric current intermittently with an integrated pulse length that is less than 10% of the operation time.

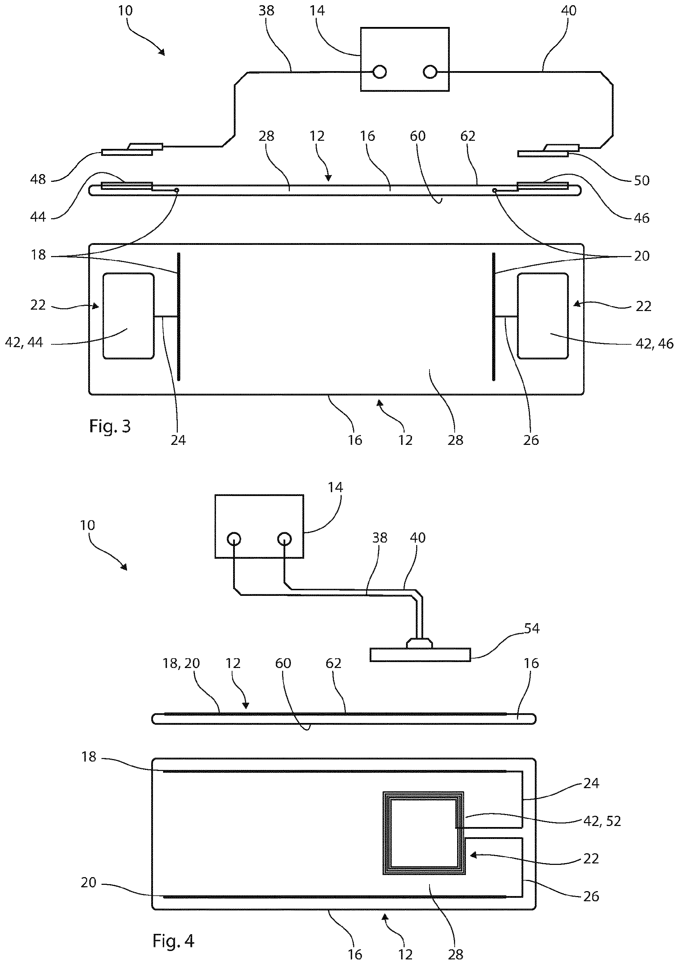

Another wound dressing system 10 is illustrated in Fig. 3. The components of this wound dressing system 10 differs from the wound dressing system 10 of Fig. 1 by the electric interface 22 being configured for a wireless power transfer from the electric power source 14.

The electric interface 22 has a receiver 42 for wireless electric power transfer. The receiver is coupled to the first electrode 18 and the second electrode 20 by the first lead 24 and the second lead 26. The receiver 42 is supported by and aligned with the first layer 16. The receiver 42 is positioned outside the wound area 28.

The receiver 42 has a flexible first receiver electrode 44 in the form of a thin metal sheath coupled to the first electrode 18 by the first lead 24. It further has a flexible second receiver electrode 46 in the form of a thin metal sheath coupled to the second electrode 20 by the second lead 26. The first receiver electrode 44 and the second receiver electrode 46 are supported by and aligned with the first layer 16.

The first and second receiver electrodes 44 and 46 can couple to a varying electric field. This way, the receiver 42 is configured to be capacitively coupled to the electric power source 14.

The system 10 has a first transmitter electrode 48 coupled to the electric power source 14 by a first electric conduit 38 in the form of a cable. The system 10 also has a second transmitter electrode 50 coupled to the electric power source 14 by a second electric conduit 40 in the form of a cable. The first and second transmitter electrodes 48 and 50 can capacitively couple to the first and second receiver electrode 44 and 46.

This way, the receiver 42 integrates the first electrode 18, the first layer 16, and the second electrode 20 in the closed electric circuit, and the receiver 42 is configured to generate an electric current in the first layer 16 by coupling to a time-varying electric field. In extension, the electric interface 22 is configured for a wireless power transfer from the electric power source 14.

Another wound dressing system 10 is illustrated in Fig. 4. The components of this wound dressing system 10 differs from the wound dressing system 10 of Fig. 3 by the first electrode 18 and the second electrode 20 contacting and connecting to the outer surface 62 instead of being embedded in the first layer 16.

The receiver 42 further has a flexible receiver coil 52 coupled to the first electrode 18 by the first lead 24 and to the second electrode 20 by the second lead 26. The receiver coil 52 is embedded in and aligned with the first layer 16. The receiver coil 52 can couple to a varying magnetic field. This way, the receiver 42 is configured to be inductively coupled to the electric power source 14.

The system 10 has a transmitter coil 54 coupled to the electric power source 14 by a first electric conduit 38 and a second electric conduit 40 in the form of cables. The transmitter coil 54 can inductively couple to the receiver coil 52. This way, the receiver 42 is configured to generate an electric current in the first layer 16 by coupling to a time-varying magnetic field.

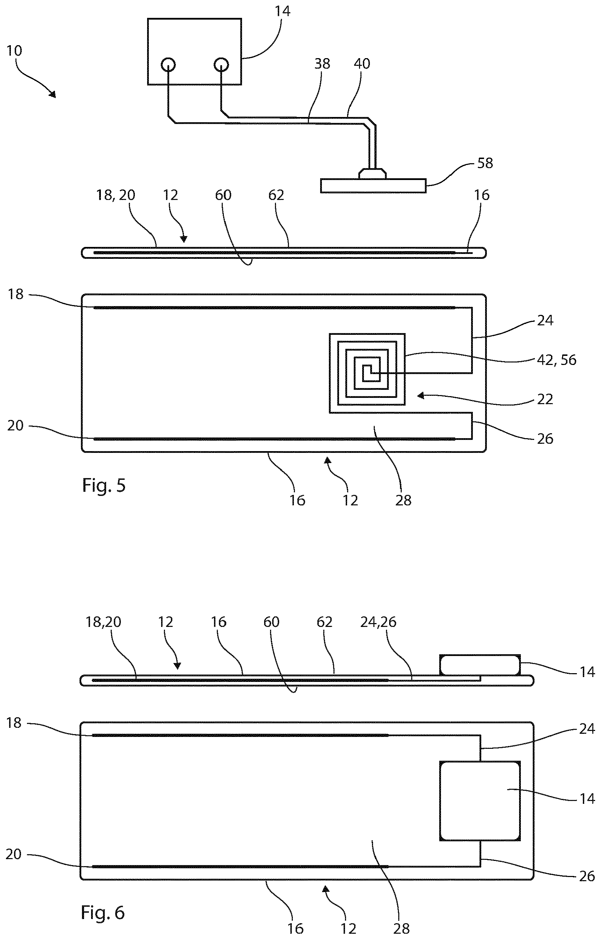

Another wound dressing system 10 is illustrated in Fig. 5. The components of this wound dressing system 10 differs from the wound dressing system 10 of Fig. 4 by the first electrode 18, the second electrode 20, and the receiver 42 being embedded in the first layer 16. Further, the receiver 42 is a flexible receiver antenna 56 coupled to the first electrode 18 by the first lead 24 and to the second electrode 20 by the second lead 26. The receiver coil 56 can couple to a varying electromagnetic magnetic field. This way, the receiver 42 is configured for radio-power transfer.

The system 10 has a transmitter antenna 58 coupled to the electric power source 14 by a first electric conduit 38 and a second electric conduit 40 in the form of cables. The transmitter antenna 58 can couple to the receiver antenna 56 for radio-power transfer. This way, the receiver 42 is configured to generate an electric current in the first layer 16 by coupling to a time-varying electromagnetic field.

In the embodiments of Figs. 3 to 5, the electric power source 14 is powered by mains electricity. It can supply an alternating current to the first layer 16 that is in the range 0.001-1 mA between the first electrode and the second electrode and at a frequency in the range 0.5 kHz and 10 kHz. It supplies the electric current intermittently with an integrated pulse length that is less than 10% of the operation time.

Another wound dressing system 10 is illustrated in Fig. 6. The components of this wound dressing system 10 differs from the wound dressing system 10 of Fig. 2 by the wound dressing 12 having no electric interface. Instead, the electric power source 14 is fixed to the first layer 16 and coupled to the first electrode 18 and the second electrode 20 by the first lead 24 and the second lead 26. The electric power source 14, the first electrode 18, the first layer 16, and the second electrode 20 form part of a closed electric circuit. This way, the electric power source 14 is configured to generate an electric current between the first electrode 18 and the second electrode 20 via the first layer 16. The electric power source 14 comprises a battery, which makes the system 10 fully portable. The electric power source 14 can supply a direct current to the first layer 16 that is in the range 0.001-1 mA at a potential difference of 0.01-5 V between the first electrode and the second electrode. It supplies the electric current intermittently with an integrated pulse length that is less than 50% of the operation time.

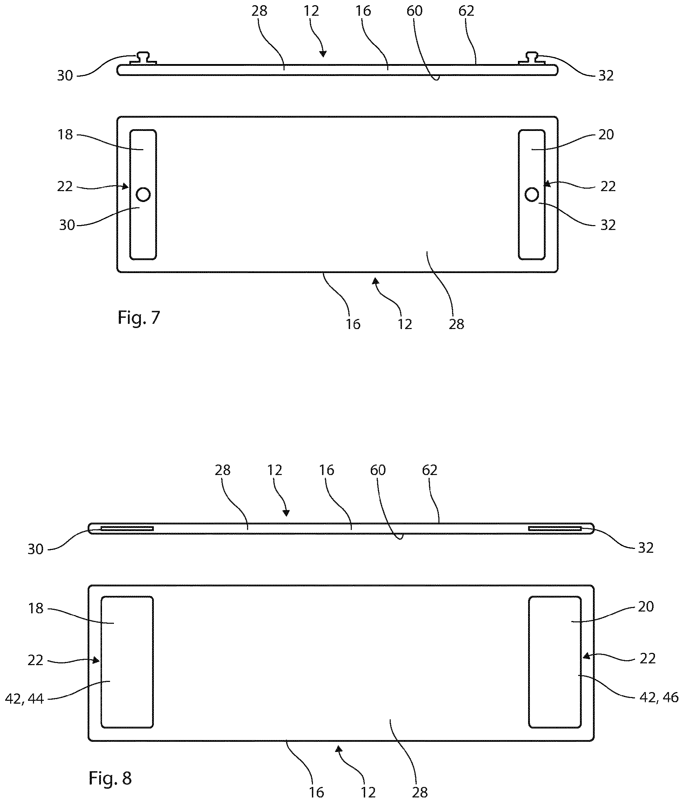

Fig. 7 illustrate alternative wound dressing 12 for the wounds systems 12 of Fig. 1. The wound dressing 12 differs by the first and second electrodes 18 and 20 being thin metal sheaths with the first fixed connector part 30 structurally integrated with the first electrode 18 and the second fixed connector part 32 structurally integrated with the second electrode 20. This way, the first and second electrodes 18 and 20 form the electric interface 22.

Fig. 8 illustrate alternative wound dressing 12 for the wounds systems 12 of Figs. 3. The wound dressing 12 differs by the first and second electrodes 18 and 20 being thin metal sheaths embedded in the first layer 16. The first electrode 18 constitutes the first receiver electrode 44, and the second electrode 20 constitutes the second receiver electrode 46. This way, the first and second electrodes 18 and 20 form the electric interface 22.

Figs. 1 to 8 show different single-layer wound dressings 12. The first layer 16 is inert to the electric current supplied by the electric power source 14. In the shown embodiments, the wound dressing 12 is an interactive, adhesive, non-absorptive, and semi-occlusive film dressing intended to be used as a primary dressing. The first layer 16 is a hydrophobic film of polyurethane with a carbon black additive making the first layer electrically conductive. In alternative embodiments, the film dressing is an interactive, occlusive, and adhesive primary or secondary dressing. The first layer 16 is a hydrophobic film of silicone rubber with a carbon black additive.

In other alternative embodiments, the wound dressing 12 is an interactive, non-adhesive, semi-occlusive and absorptive foam dressing intended to be used as a primary or secondary dressing. The first layer 16 is composed of a hydrophilic polyurethane foam with a carbon black additive making the first layer electrically conductive. In other alternative embodiments, the wound dressing 12 is a passive, non-adhesive, and non-occlusive, gauze or tulle dressing intended to be used as a primary or secondary dressing. The first layer 16 is composed of woven or non-woven cotton or rayon fibers with carbon fibers added to make the first layer 16 electrically conductive.

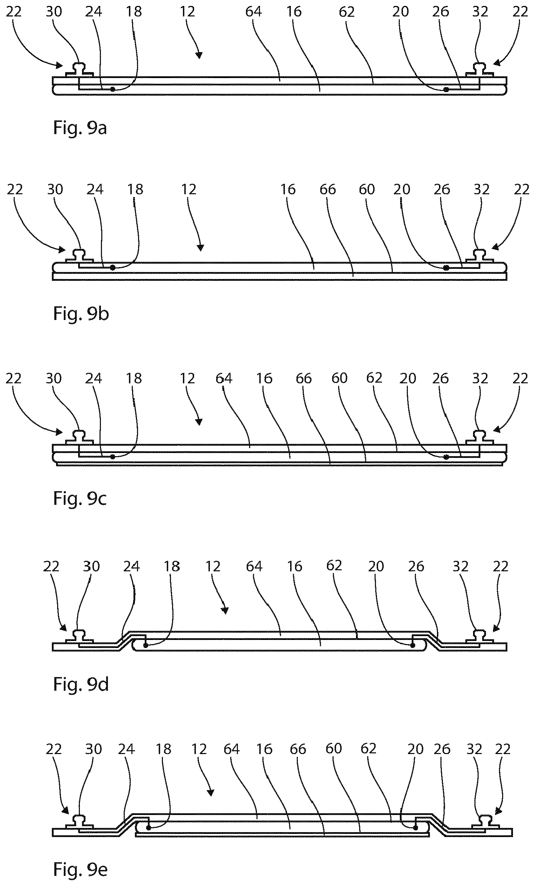

Figs. 9a to 9e show embodiments of different multi-layer wound dressings 12. The electric interface 22 corresponds to that described in relation to Fig. 1. The first layer 16 is inert to the electric current supplied by the electric power source 14. In other embodiments (not shown), the electric interface 22 and the first and second electrodes 18 and 20 correspond to those described in relation to Fig. 2.

In the embodiment of Fig. 9a, the first layer 16 is composed of a hydrophilic polyurethane foam with a carbon black additive making the first layer 16 electrically conductive. The wound dressing 12 further has outer additional layer 64 that is attached to and covers the outer surface 62 of the first layer 16. The shape and area of the outer additional layer 64 matches the shape of the first layer 16. The outer additional layer 64 is composed of an electrically insulating hydrophobic foam of silicone rubber. The wound dressing 12 is interactive, adhesive, and semi-occlusive an intended to be used as a primary dressing. The electric interface 22 is supported by the outer additional layer 64.

In the embodiment of Fig. 9b, the first layer 16 is composed of a hydrophilic polyurethane foam with a carbon black additive. The wound dressing 12 further has inner additional layer 66 that is attached to and covers the inner surface 60 of the first layer 16. The shape and area of the inner additional layer 66 matches the shape of the first layer 16. The inner additional layer 66 is composed of electrically insulating and hydrophilic polyurethane rubber. The wound dressing 12 is interactive, adhesive, and semi-occlusive an intended to be used as a primary dressing. The electric interface 22 is supported by the first layer 16.

In the embodiment of Fig. 9c, the first layer 16 is composed of non-woven rayon fibers with carbon fibers added to make the first layer 16 electrically conductive. The wound dressing 12 further has outer additional layer 64 that is attached to and covers the outer surface 62 of the first layer 16. The shape and area of the outer additional layer 64 matches the shape of the first layer 16. The outer additional layer 64 is composed of an electrically insulating semi-occlusive polyurethane film. The wound dressing 12 further has inner additional layer 66 that is attached to and covers the inner surface 62 of the first layer 16. The shape and area of the inner additional layer 66 matches the shape of the first layer 16. The inner additional layer 66 is composed of an electrically insulating porous-polymer coating. The wound dressing 12 is passive, non-adhesive, and semi-occlusive and intended to be used as a primary dressing. The electric interface 22 is supported by the outer additional layer 64.

In the embodiment of Fig. 9d, the first layer 16 is composed of woven cotton fibers with carbon fibers added to make the first layer 16 electrically conductive. The wound dressing 12 further has outer additional layer 64 that is attached to and covers the outer surface 62 of the first layer 16. The shape of the outer additional layer 64 matches the shape of the first layer 16, but its area is greater and the first layer 16 extends from the first layer 16. The first layer 16 is non-adhesive and the outer additional layer 64 is adhesive, and the outer layer 64 attaches the wound dressing 12 relative at the wound. The outer additional layer 64 is composed of electrically insulating, perforated, and coated paper. The wound dressing 12 is passive, adhesive, and non-occlusive and intended to be used as a secondary dressing. The first fixed connector part 30 and the second fixed connector part 32 are supported by the outer additional layer 64 and are tangentially spaced apart from the first layer 16. This means that the electric interface 22 is spaced apart from the wound area.

In the embodiment of Fig. 9e, the wound dressing 12 has the same layers as the embodiment of 9c, but with the difference that the outer additional layer 64 has greater area than and extends from the first layer 16. The first layer 16 is non-adhesive and the outer additional layer 64 is adhesive, and the outer layer 64 attaches the wound dressing 12 relative at the wound. The wound dressing 12 is passive, adhesive, and semi-occlusive and intended to be used as a primary dressing. The first fixed connector part 30 and the second fixed connector part 32 are supported by the outer additional layer 64 and are tangentially spaced apart from the first layer 16. This means that the electric interface 22 is spaced apart from the wound area.

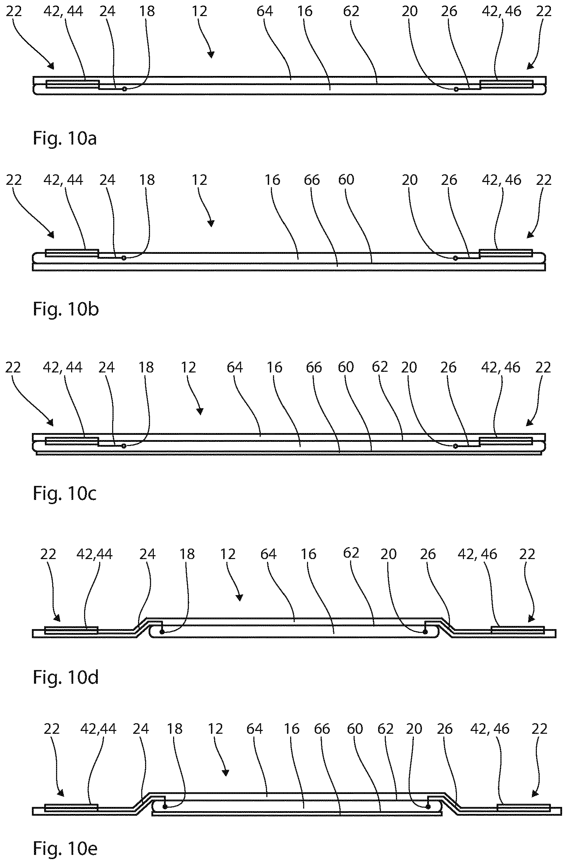

Figs. 9a to 9e show embodiments of different multi-layer wound dressings 12. The electric interface 22 corresponds to that described in relation to Fig. 3. In other embodiments (not shown), the electric interface 22 and the first and second electrodes 18 and 20 correspond to those described in relation to Figs. 4 to 6. The respective wound dressings 12 in Figs. 10a to 10e has the same layers as in Figs. 9a to 9e. In the embodiments of Figs 9d and 9e, the first receiver electrode 44 and the second receiver electrode 46 are supported by the outer additional layer 64 and are tangentially spaced apart from the first layer 16. This means that the electric interface 22 is spaced apart from the wound area.

### Item list

10 wound dressing system
12 wound dressing
14 electric power source
16 first layer
18 first electrode
20 second electrode
22 electric interface
24 first lead
26 second lead
28 wound area
30 first fixed connector part
30' fixed connector part
32 second fixed connector part
34 first detachable connector part
34' detachable connector part
36 second detachable connector part
38 first electric conduit
40 second electric conduit
42 receiver
44 first receiver electrode
46 second receiver electrode
48 first transmitter electrode
50 second transmitter electrode
52 receiver coil
54 transmitter coil
56 receiver antenna
58 transmitter antenna
60 inner surface
62 outer surface
64 outer additional layer
66 inner additional layer

## Claims

**1.** A wound dressing (12) for covering a wound, wherein the wound dressing (12) comprises:
- a first electrode (18),
- a second electrode (20),
- a first layer (16) for placement on top of a wound, and
- an electric interface (22),
wherein the first electrode (18) contacts the first layer (16),
the second electrode (20) contacts the first layer (16) and is spaced apart from the first electrode (18),
the first layer (16) is electrically conductive and constitutes an uninterrupted resistive conductor between the first electrode (18) and the second electrode (20),
the electric interface (22) is coupled to the first electrode (18) and the second electrode (20) and configured to operationally couple to an electric power source (14) for generating an electric current between the first electrode (18) and the second electrode (20) via the first layer (16).

**2.** The wound dressing (12) according to claim 1, wherein the first layer (16) is inert to an electric current passing through the first layer (16).

**3.** The wound dressing (12) according to claim 1 or 2, wherein the first layer (16) defines a wound area (28) for covering the wound, the wound area (28) has a first side and an opposite second side, and the first electrode (18) is positioned at the first side and the second electrode (20) is positioned at the second side.

**4.** The wound dressing (12) according to any of the claims 1 to 3, wherein first layer (16) has an inner surface (60) and an outer surface (62), and the first electrode (18) and the second electrode (20) contact the outer surface (62) of the first layer (16).

**5.** The wound dressing (12) according to any of the claims 1 to 3, wherein first layer (16) has an inner surface (60) and an outer surface (62), and the first electrode (18) and the second electrode (20) are embedded in the first layer (16) between the first inner surface (60) and the outer surface (62) .

**6.** The wound dressing (12) according to any of the claims 1 to 5, wherein the wound dressing (12) is a single-layer dressing.

**7.** The wound dressing (12) according to claim 6, wherein the complete first layer (16) is adhesive.

**6.** The wound dressing (12) according to any of the claims 1 to 5, wherein the wound dressing (12) further comprises:
- an outer additional layer (64) attached to the first layer (16), wherein the outer additional layer (64) is electrically insulating.

**7.** The wound dressing (12) according to claim 6, wherein the outer additional layer (64) is an adhesive backing layer extending from the first layer (16).

**8.** The wound dressing (12) according to any of the claims 1 to 7, wherein the wound dressing (12) further comprises:
- an inner additional layer (66) attached to the first layer (16), wherein the inner additional layer (66) is electrically insulating.

**9.** The wound dressing (12) according to any of the claims 1 to 8, wherein the electric interface (22) is configured for a wired power transfer from the electric power source (14), and the electric interface (22) comprises a first fixed connector part (30) and a second fixed connector part (32), wherein each of the first fixed connector part (30) and the second fixed connector part (32) is a single-terminal connector part, the first fixed connector part (30) is coupled to the first electrode (18) by a first lead (24) and the second fixed connector part (32) is coupled to the second electrode (20) by a second lead (26), the first layer (16) defines a wound area (28) for covering the wound, and the first fixed connector part (30) the and second fixed connector part (32) are spaced apart from the above-mentioned wound area (28).

**10.** The wound dressing (12) according to any of the claims 1 to 8, wherein the electric interface (22) is configured for a wired power transfer from the electric power source (14), and the electric interface (22) comprises a single fixed connector part (30'), wherein the single fixed connector part (30') is a two-terminal connector part, wherein the fixed connector part (30') is coupled to the first electrode (18) by a first lead (24) and to the second electrode (20) by a second lead (26), the first layer (16) defines a wound area (28) for covering the wound, and the fixed connector part (30') is spaced apart from the wound area (28).

**11.** The wound dressing (12) according to any of the claims 1 to 8, wherein the electric interface (22) is configured for a wireless power transfer from the electric power source (14), the electric interface (22) comprises a receiver (42) for wireless electric power transfer, and the first electrode (18) and the second electrode (20) form part of the receiver (42) .

**12.** The wound dressing (12) according to any of the claims 1 to 8, wherein the electric interface (22) is configured for a wireless power transfer from the electric power source (14), wherein the electric interface (22) comprises a receiver (42) for wireless electric power transfer, the receiver (42) is coupled to the first electrode (18) by a first lead (24) and to the second electrode (20) by a second lead (26), and the receiver (42) is positioned between the first electrode (18) and the second electrode (20).

**13.** A wound dressing system (10), wherein the wound dressing system (10) comprises:
- a wound dressing (12) according to any of the claims 1 to 12, and
- an electric power source (14) operationally coupled to the electric interface (22) and configured for generating an electric current between the first electrode (18) and the second electrode (20) via the first layer (16) for inhibiting, or reducing, microbiological, or bacterial, growth in, and/or on, the first layer (16).

**14.** A method for reducing microbiological growth in and/or on a wound dressing (12) according to claim 1, wherein the method comprises:
- generating an electric current in the first layer (16).
